# FASCICULE DE BREVET EUROPEEN

(11) **EP 1 114 348 B1**
(45) Date de publication et mention de la délivrance du brevet: **27.11.2002**
(21) Numéro de dépôt: 99942971.5
(22) Date de dépôt: 15.09.1999
(51) Int. Cl.: G02B 23/26, A61B 1/04

(54) **DISPOSITIF D'OBSERVATION DE L'INTERIEUR D'UN CORPS PRODUISANT UNE QUALITE D'OBSERVATION PERFECTIONNEE**
Vorrichtung zur Beobachtung des Inneren eines Körpers mit verbesserter Beobachtungsqualität
DEVICE FOR OBSERVATION INSIDE A BODY PROVIDING IMPROVED QUALITY OF OBSERVATION

(30) Priorité: 15.09.1998 FR 9811486
(43) Date de publication de la demande: 11.07.2001
(73) Titulaire: ASSISTANCE PUBLIQUE, HOPITAUX DE PARIS, 75004 Paris (FR); INSTITUT NATIONAL DE LA SANTE ET DE LA RECHERCHE MEDICALE (INSERM), 75013 Paris (FR)
(72) Inventeur: LE GARGASSON, Jean-François, F-94350 Villiers s/Marne (FR); LAMARQUE, Frédéric, F-78690 Les Essarts le Roi (FR); CHADUC, Jean-Paul, F-63000 Clermont-Ferrand (FR)
(74) Mandataire: Pontet, Bernard
(86) Numéro de dépôt international: FR9902188
(87) Numéro de publication internationale: WO00016151

(56) Documents cités:
- DE-A- 4 207 092
- US-A- 5 074 642
- US-A- 5 103 497
- US-A- 5 200 838
- US-A- 5 280 378
- US-A- 5 425 123
- PATENT ABSTRACTS OF JAPAN vol. 011, no. 103 (P-562), 2 avril 1987 (1987-04-02) & JP 61 251819 A (OLYMPUS OPTICAL CO LTD), 8 novembre 1986 (1986-11-08)
- PATENT ABSTRACTS OF JAPAN vol. 016, no. 221 (E-1205), 22 mai 1992 (1992-05-22) & JP 04 038092 A (TOSHIBA CORP;OTHERS: 01), 7 février 1992 (1992-02-07)

## Description

La présente invention concerne les dispositifs d'observation de l'intérieur d'un corps, vivant ou manufacturé, comportant un canal souple de transmission d'images depuis l'intérieur du corps jusqu'à un dispositif de présentation d'images qui est extérieur au corps. Dans de tels dispositifs, la souplesse du canal est mise à profit pour parcourir une partie du corps, reliant une zone à observer intérieure au corps, à un poste d'observation extérieur.

La souplesse du canal doit être suffisante pour autoriser le canal à pénétrer dans des zones du corps qui peuvent être particulièrement enchevêtrées.

De manière habituelle, le canal doit également être suffisamment rigide pour permettre de guider, depuis l'extérieur du corps, par action sur le canal, une extrémité d'exploration de celui-ci.

Dans de nombreux dispositifs de ce type, le canal est manipulé pour faire atteindre à une extrémité d'illumination de ce canal, adjacente au corps, une zone à observer intérieure au corps qui est inaccessible directement depuis l'extérieur du corps avec des radiations photoniques.

De tels dispositifs constituent donc de manière habituelle des moyens d'exploration de parties inaccessibles à l'observation directe.

Lorsque le corps à observer est le corps d'un être vivant, de tels dispositifs sont communément appelés « endoscopes ».

Les endoscopes connus sont souvent basés sur un principe d'éclairement global d'un champ, en lumière poly- ou monochromatique. Un éclairage est transmis par une voie d'illlumination, et l'information visuelle est transmise sur un autre canal.

Ces endoscopes utilisent pour l'observation une surface sensible photographique ou une caméra CCD. Ils nécessitent par là des niveaux d'éclairement élevés.

Leur niveau de performance est essentiellement défini par la résolution optique d'un système pour véhiculer les images et par la qualité d'un système optique. Ils ne permettent pas de faire des observations avec une résolution en profondeur satisfaisante et ne permettent pas non plus d'obtenir un contraste acceptable, notamment dans les milieux diffusants.

De plus ces dispositifs ne permettent pas d'obtenir simultanément un grand champ d'observation et une large ouverture numérique dans un espace réduit.

On a également proposé, dans le document US 5 074 642, un endoscope comportant un faisceau de fibres dont l'extrémité côté observateur est placée face à une source lumineuse et entraînée en vibration face à cette source lumineuse les différentes fibres sont balayées par la source et qu'on obtient ainsi un balayage d'une zone du corps.

Un tel dispositif ne permet qu'un balayage très lent des fibres et s'avère notamment trop lent pour obtenir une image vidéo en temps réel.

Dans le domaine de la microscopie, on a proposé des dispositifs appelés « microscopes confocaux ». Ces dispositifs ne permettent pas de réaliser des examens endoscopiques. Les microscopes confocaux servent à différents types d'examen microscopique, comme l'observation de coupes par transillumination, ou encore l'observation anatomique directe par rétro-illumination, ou l'exploration par le truchement de colorants comme la fluorescéine, le verre d'indocianine ou l'orange d'acrédine.

Avec certains appareils expérimentaux récents, les explorations fonctionnelles sont également possibles comme l'étude d'activités fonctionnelles des voies cérébrales ou l'évaluation de l'état de parois vasculaires. Cependant, l'observation de l'activité neurologique cérébrale par exemple, ne peut être réalisée que sur l'animal, car il est nécessaire de faire une ablation de la calotte crânienne pour placer un microscope confocal d'observation.

DE 4 207 092 décrit un endoscope basé sur le principe d'un éclairement global du champ à observer.

Le document US 5,200,838 décrit un endoscope utilisant un éclairement à balayage à trames.

Un but de la présente invention est de proposer un dispositif d'observation de l'intérieur d'un corps, tel que le corps d'un être vivant, comprenant des moyens d'illumination de l'intérieur du corps, un moyen de présentation d'une image, et un canal flexible destiné à parcourir une partie intérieure du corps et à acheminer une image, depuis l'intérieur du corps jusqu'au moyen de présentation, qui fournisse une image particulièrement nette d'une zone d'observation intérieure au corps (par des techniques confocales ou interférométriques).

Un autre but de la présente invention est de proposer un dispositif de type endoscope fournissant une résolution en profondeur et un contraste acceptable, y compris dans des milieux à observer diffusant.

Un autre but de l'invention est de proposer un endoscope à haute sensibilité photonique.

Un autre but est de proposer un dispositif autorisant une séparation spatiale et temporelle du flux parasite lié à l'éclairement de celui correspondant à la réflexion tissulaire.

Un autre but est de proposer un dispositif limitant la réflexion dans des milieux diffusants sus et sous-jacents d'un milieu tissulaire étudié.

Un autre but est de proposer un dispositif permettant d'éliminer des réflexions liées à des élément optiques de montage.

L'invention vise également à proposer un dispositif permettant de réaliser une image de tissu avec un faisceau lumineux d'exploration de large ouverture en plaçant le dispositif optique au contact ou à quelques millimètres des tissus ou des surfaces à étudier.

Selon un autre but, l'invention propose un dispositif robuste à la vibration éventuelle du montage et ne nécessitant pas d'entretien ni de réglage particulier.

Un autre but de l'invention est de fournir un dispositif pouvant fournir une résolution satisfaisante avec un groupe de fibres de faible diamètre.

L'invention vise en outre à fournir un dispositif d'observation ne présentant dans sa partie adjacente au corps observé aucune partie électronique ou mécanique qui soit susceptible d'être attirée ou endommagée lorsque soumise à un champ magnétique intense, afin de pouvoir être utilisé conjointement à un dispositif d'observation à résonance magnétique.

Elle vise également à permettre un balayage de fibres optiques qui soit particulièrement rapide.

Ces différents buts sont atteints grâce à un dispositif d'observation de l'intérieur d'un corps tel que défini dans la revendieation 1.

D'autres caractéristiques, buts et avantages de l'invention apparaîtront à l'examen de la description détaillée ci-dessous et de dessins annexés sur lesquels :
- la figure 1 est une vue d'ensemble sous forme de blocs fonctionnels d'un mode de réalisation d'un dispositif selon l'invention ;
- la figure 2 est une vue plus détaillée d'un endoscope conforme au schéma de la figure 1.

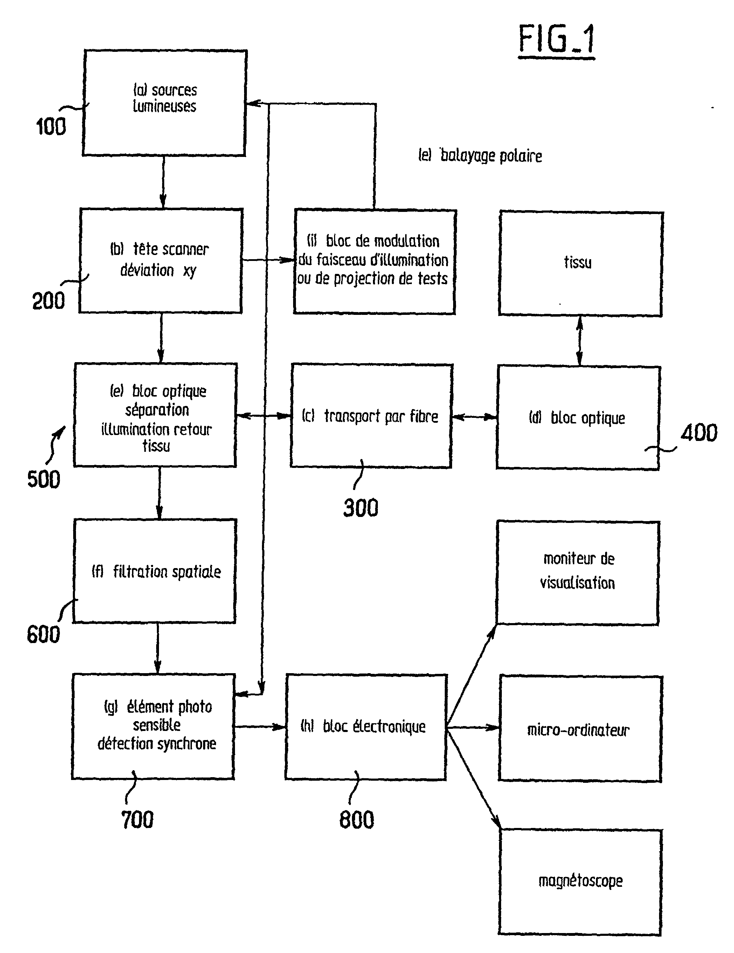

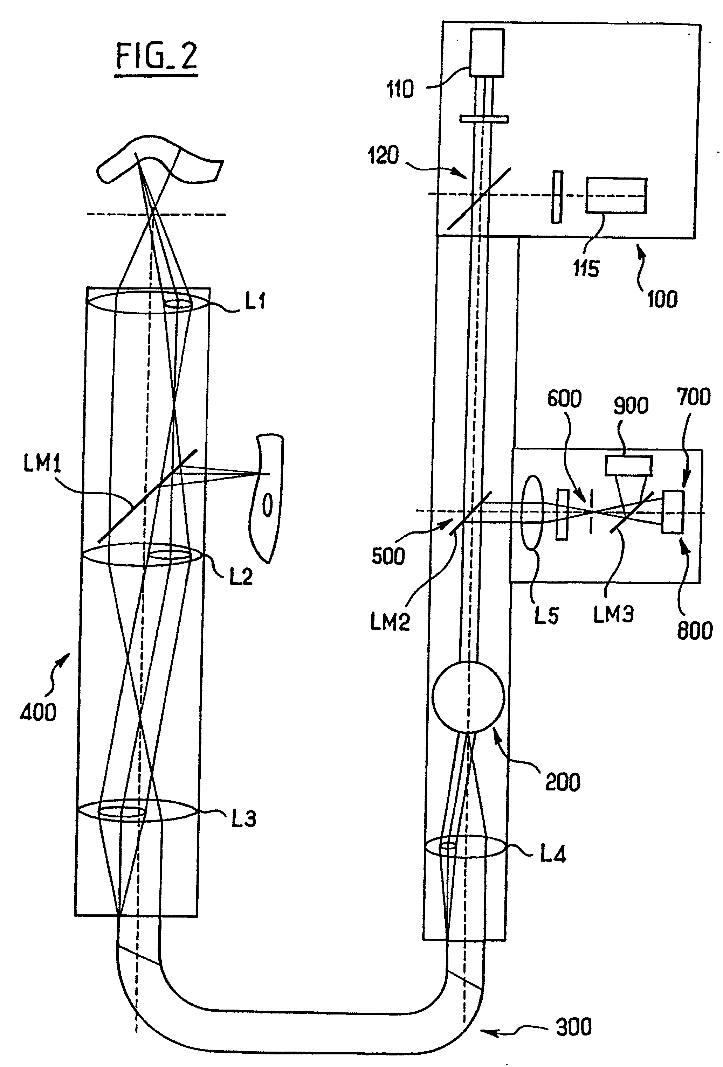

Le dispositif des figures 1 et 2 comporte huit parties principales : un bloc de source lumineuse 100, un bloc de balayage 200, un module de transport d'images 300, un bloc optique avant 400, un bloc optique arrière 500, un bloc de filtration spatiale 600, un bloc de transformation de signal optique en signal électrique 700, un bloc électronique 800.

Le bloc source lumineuse 100 produit une lumière par l'intermédiaire d'une source laser de faible puissance 110 et par une source de lumière visible 115. L'éclairement peut être réalisé par un faisceau laser de longueur d'onde visible ou infrarouge.

Ces deux sources ont des directions d'émission perpendiculaires l'une à l'autre, un miroir dichroïque 120 étant placé à 45° de la direction d'émission de la source de lumière visible pour dévier celle-ci selon une direction parallèle à la direction d'émission de la source laser 110. Ce miroir 120 est choisi pour n'avoir aucun effet déviateur sur les rayons émis par la source laser 110. Ces deux sources peuvent être des sources de type lampe arc xénon ou encore par des sources à incandescence.

Le choix de l'assemblage et des caractéristiques des sources utilisées dépend du type d'application. Ainsi, les courtes longueurs d'onde et les radiations infrarouges permettent-elles d'exciter respectivement la fluorescéine et le verre d'indoscianine. Les faisceaux infrarouges autorisent aussi l'observation des couches tissulaires profondes alors que les longueurs d'ondes visibles mettent bien en évidence les couches les plus superficielles.

L'intensité des différentes sources est choisie pour être compatible avec les règles de sécurité concernant la destruction des tissus biologiques, c'est à dire notamment la norme AFNOR C43-801, 1992 et la norme ANSI Z136.1, 1993.

Le dispositif source décrit ici permet le mélange des deux sources laser et visible ainsi que la réglage de la puissance du faisceau de chacune d'elles. Devant chacune de ces deux sources 110 et 115 est placé un modulateur respectif commandé par un dispositif électronique ou optique permettant de réaliser des trains d'impulsions lumineuses qui permettront une détection synchrone d'un flux réfléchi en s'affranchissant des réflexions, comme cela sera décrit par la suite. Des trains d'impulsions peuvent être générés par une commande directe des éléments d'illumination et de détection.

Dans l'exemple particulier décrit à la figure 2, on réalise une illumination tissulaire en infrarouge. Le flux d'illumination est généré par une diode à laser infrarouge émettant à une longueur d'onde située autour de 800 nm.

Plus généralement le bloc source lumineuse 100 peut être composé de une ou de plusieurs sources laser et/ou de source de lumière monochromatique incohérente et/ou polychromatique incohérente. En aval de ce bloc source lumineuse, par rapport au trajet de la lumière émise, se trouve le bloc de balayage 200 consistant en un déviateur des rayons lumineux émis par la source 100.

En aval de ce bloc de balayage est disposée une lentille L4 suivie d'un groupe de fibres optiques 300 flexibles et parallèles entre elles. Le groupe de fibres optiques 300 ou guide d'images présente les extrémités de ses fibres adjacentes à la lentille L4 dans un plan parallèle à cette lentille.

Le bloc de balayage 200 est constitué d'éléments optiques qui sont dans l'exemple de réalisation particulier décrit ici des éléments optiques acousto-optiques. Selon une variante de l'invention, ces éléments peuvent être des éléments mécano-optiques.

L'ensemble formé du bloc de balayage 200 et de la lentille L4 a pour rôle de dévier le faisceau issu de la source lumineuse 100 dans des directions horizontales et verticales, afin de balayer la section d'entrée du guide d'images à fibres optiques 300.

Le bloc de balayage 200 réalise donc des déviations angulaires successives du faisceau d'illumination provenant de la source 100. Ces déviations angulaires sont transformées par la lentille L4 en une déviation latérale focalisée sur la section d'entrée du guide d'images à fibres 300, produisant ainsi un éclairement successif des différentes fibres optiques du groupe de fibres 300. Ainsi, à chaque fibre optique correspond un angle de déviation du faisceau d'illumination et un seul en sortie du module de balayage 200.

Chaque faisceau dévié angulairement est redirigé par la lentille L4 en un faisceau ayant une direction constante par rapport à un axe principal d'entrée du guide d'image 300, ici sensiblement parallèle à l'axe principal d'entrée du guide d'image 300.

Un tel faisceau redirigé selon une direction choisie sensiblement constante, parallèle ou non à l'axe principal d'entrée du groupe de fibres, a, au cours du balayage, un positionnement latéral par rapport à l'axe principal d'entrée du guide qui correspond à une fibre choisie du groupe de guides.

On obtient donc en aval de la lentille L4 un balayage latéral à l'axe d'entrée du guide, c'est à dire un déplacement latéralement à l'axe d'entrée du guide d'un faisceau ayant une direction constante, ici une direction parallèle à cet axe.

Ce balayage latéral est obtenu par l'action de la lentille L4 à partir d'un balayage angulaire amont, un tel balayage angulaire étant mis en oeuvre avec un matériel peu encombrant et particulièrement rapide.

Le fait de rediriger les rayons selon une direction constante peut être également réalisé par un ensemble optique de type objectif de microscope. On réalise ainsi par coopération du bloc de balayage 200 et de la lentille L4 un système optique apte à former un point de focalisation dans un plan d'interface d'entrée du guide d'images 300.

La lentille L4 permet donc de transformer le balayage angulaire du faisceau d'illumination en un balayage en translation, afin d'attaquer les fibres optiques sous un angle constant, normal à leurs sections.

Les fibres optiques sont donc adressées au cours du temps de telle sorte qu'à un instant donné une, voire un ensemble de fibres, transportent le flux d'illumination à l'autre extrémité du guide d'images à fibre optique.

Plus précisément encore, dans l'exemple de réalisation décrit ici, le faisceau émanant de la source 100, qui est collimatée, est dévié en X et Y par le bloc 200 qui est un dispositif opto-mécanique composé d'un galvanomètre asservi et d'un oscillateur résonant à 8 kHz. La lentille L4 présente une longueur focale de 2,8 mm.

Dans le cas d'un éclairage polychromatique, la déviation optique doit être réalisée par un système opto-mécanique. Dans le cas d'utilisation d'un faisceau laser ou d'une source incohérente monochromatique pour illumination, la déviation optique peut être également réalisée par un déflecteur acousto-optique bidimensionnel situé à une distance convenable pour assurer le balayage de toute la surface de l'interface d'entrée du guide d'images à fibre optique.

Si les sources utilisées sont des sources incohérentes monochromatiques, la déviation peut être obtenue grâce à un déflecteur acousto-optique bidimensionnel ou à deux déflecteurs acousto-optiques monodimensionnel. Un tel dispositif présente l'avantage de ne pas comporter de pièces mécaniques en mouvement. Les déflecteurs acousto-optiques sont sensibles à la longueur d'ondes. Il conviendra donc dans de tels dispositifs de disposer d'un déflecteur pour chaque longueur d'onde utilisée.

Lorsque les déviateurs utilisés sont des déviateurs mécano-optiques, la déviation horizontale peut être obtenue soit à l'aide d'un miroir résonant optomécanique, soit grâce à un moteur tournant équipé d'un miroir polygonal à facettes, soit encore par un miroir galvanométrique. La déviation verticale peut être obtenue grâce à un dispositif galvanométrique comportant un axe équipé d'un miroir.

Ce dispositif permet donc de former une image de la source qui est inférieure ou égale en diamètre, au diamètre de la section d'une fibre optique, dans le plan de l'interface d'entrée du guide d'images à fibres optiques 300.

L'ensemble formé de la source, du bloc 200 et de la lentille L4 peut être remplacé par une matrice photo-émissive active. Une telle matrice peut être à commande séquentielle électrique ou photonique. Le bloc source 100 peut être, dans certaines dispositions, couplé directement au guide d'images à fibres optiques (notamment dans le cas d'un laser thérapeutique).

Le guide d'images 300 est constitué d'un groupe de fibres optiques ordonnées dans le même ordre aux interfaces d'entrée et de sortie de ce groupe de fibres. Au sein du groupe, les fibres optiques sont ordonnées selon un ordre de rangement qui est identique aux deux extrémités du guide d'image.

Ainsi le balayage du groupe de fibres effectué à l'extrémité côté observateur se retrouve selon un même trajet transversal aux fibres à l'extrémité côté corps du groupe de fibres.

La lumière étant prélevée, côté observateur, sur les fibres successives au cours de ce balayage, le balayage de prélèvement de lumière suit lui aussi le même trajet transversal que le balayage de la zone.

Les moyens d'illumination et de prélèvement situés côté observateur du groupe de fibres illuminent et prélèvent la lumière sur l'extrémité du groupe de fibres comme s'il s'agissait de la zone du corps à observer, de sorte que le groupe de fibres, par son caractère ordonné, produit au vu de ces moyens un transport de l'image, du corps jusqu'à l'extrémité du guide adjacente à ces moyens et chaque point de cette image, c'est à dire craque fibre a la même position dans l'image transportée à l'entrée comme à la sortie du guide.

Dans l'art antérieur, les dispositifs d'observation proposés ne font généralement appel qu'à un ensemble de fibres sans correspondance parfaite entre la surface du champ imagé et la surface transversale de ce que l'on appelle alors un faisceau de fibres, même au grossissement optique près.

Dans le mode de réalisation décrit ici, la surface de l'interface de sortie du guide d'images à fibres optiques est égale au champ d'observation ou surface imagée du tissu observé, au grossissement optique près d'un système optique placé entre l'interface de sortie du guide d'images à fibres optiques et le tissu observé.

Dans le cas décrit ici, le guide d'images est constitué par exemple d'un groupe de 10 000 fibres réparties selon un carré ayant un côté de 100 fibres. On pourra également réaliser un guide d'images ayant par exemple un carré de 800 x 600 fibres, ou plus.

Plus précisément, le guide d'images à fibres optiques est constitué de 100 x 100 fibres optiques jointives de 4,5 µm de diamètre et de 0,35 d'ouverture numérique. Ce guide d'images à fibres optiques a ici une longueur de 10 mètres. La valeur minimum recommandé de 2 mètres permet de ne pas avoir d'effet de cône d'ombre à la sortie de la fibre.

L'extrémité de la fibre optique dirigée vers le corps à observer est disposée dans le plan focal d'une lentille L3, appartenant au bloc optique avant 400.

Le bloc optique avant 400 comprend l'extrémité de sortie du guide d'images à fibres optiques 300, puis, le long d'un parcours de la lumière se propagent vers le corps à observer, successivement une première lentille L3, une seconde lentille L2, une lame LM1 et une lentille L1.

L'extrémité de sortie du guide d'images est montée sur le bloc optique 400, le bloc étant de préférence mobile par rapport à cette interface de sortie des guides.

Comme on l'a décrit précédemment, le balayage optique du groupe de fibres par le bloc 200 a pour effet d'illuminer successivement chacune des fibres optiques du groupe 300. La sortie de chaque fibre va donc former une source lumineuse secondaire à un instant donné, et cette source lumineuse se déplace linéairement d'une fibre à l'autre.

Cette source lumineuse secondaire se trouvant dans le plan focal de la lentille L3, ce déplacement transversal de la source lumineuse secondaire est transformé en une déviation angulaire par la lentille L3. La lentille L2 et la lentille L3 présentent un foyer en commun situé entre ces deux lentilles, de sorte que la déviation angulaire en sortie de la lentille L3 est transformée à nouveau par la lentille L2 en une déviation transversale.

La lentille L1 dévie alors ce faisceau en un faisceau focalisé sur un point du plan conjugué au plan focal de la lentille L2. La lentille L1 focalise le faisceau dans le plan du corps à observer. Ce point de concentration du faisceau de sortie parcourt un plan d'illumination du corps à observer lorsque les fibres du groupe de fibres sont successivement illuminées. Le flux lumineux réfléchi par chaque point du corps illuminé forme en passant dans la lentille L1 une image ponctuelle sur la fibre d'illumination par le truchement des lentilles L2 et L3. En conséquence, chaque fibre optique joue successivement le rôle d'un filtre spatial.

Les moyens 400 sont donc prévus pour faire correspondre aux différentes fibres du groupe de fibres 300 des positions différentes du point focal, selon une relation telle que le point de concentration effectue un balayage de la zone à observer lorsque les fibres sont balayées par le dispositif d'illumination.

Dans l'exemple particulier de réalisation décrit ici, la lentille L3 présente une distance focale de 2,8 mm. Elle collimate le faisceau de sortie de la fibre et transforme le balayage en translation de fibre à fibre en un balayage angulaire du faisceau d'illumination, dont le centre de rotation est situé au plan focal du système afocal L2-L3. Les longueurs focales calculées pour L3 et L2 permettent de balayer un champ tissulaire à observer de 10° x 10° par une tâche d'illumination de l'ordre de 2 µm. La mise au point du plan d'observation dans le plan tissulaire que l'on désire observer est obtenue en déplaçant l'extrémité de la fibre optique par rapport à la lentille L3.

Les déplacements du dispositif constitué par les trois lentilles L1, L2 et L3, ou de certaines lentilles particulières de ce dispositif, permettent plus généralement de faire varier la position du plan d'illumination confocal du tissu. L'ouverture de la fibre optique, et les longueurs focales respectives des lentilles du bloc optique 400, conditionnent l'ouverture du faisceau d'illumination et la valeur du champ tissulaire exploré. Le bloc 400 est donc mobile et interchangeable par rapport au système de transport d'images.

Du côté des fibres qui est adjacent au corps, entre les lentilles L1 et L2, est disposée une lame LM1. Cette lame LM1 est une lame de renvoi disposée à 45°, réfléchissant une série de longueurs d'ondes latéralement au bloc 400 et permettant une observation latérale de coupes de tissus. La lentille L1 peut être un ensemble de lentilles permettant d'élargir le champs d'observation donné par l'ensemble L3 et L2 de lentilles ou de groupes de lentilles, la lame LM1 permet d'obtenir simultanément des vues axiales et latérales de champ d'observation et de grandissement respectivement différents.

On choisit préférentiellement une lame LM1 sensible au chromatisme et pouvant être changée en fonction de l'usage et des longueurs d'ondes utilisées pour une telle visualisation latérale.

Un tel dispositif permet d'obtenir simultanément une vue axiale et une vue latérale.

Dans un dispositif le plus avantageux, la lame LM1 réfléchit les infrarouges et une longueur d'onde visible, à 45°, et laisse passer le reste du spectre selon l'axe du bloc 400, en direction du guide d'images.

La lame LM1 réfléchit à 45° les infrarouges (830 nm, 800 nm) et une ou plusieurs longueurs d'ondes visibles (par exemple les longueurs d'ondes de 680 nm, 514 nm, 477 nm). Cette lame LM1 laisse passer les autres longueurs d'ondes. La lame LM1 permet donc d'observer des images latéralement avec un petit champ et une grande résolution et des images axiales avec un grand champ et une résolution moindre.

Ainsi, dans ce dispositif, l'un des dispositifs de présentation d'images est apte à fournir une vue d'une zone générale et l'autre à fournir une vue microscopique confocale d'un zone plus restreinte comprise ou non dans la zone générale.

Les deux dispositifs de présentation d'images sont de plus aptes à fournir simultanément une image microscopique et une image macroscopique.

Le montage décrit précédemment autorise une mise au point dans différents plans de tissus observés. Une telle mise au point de la position du plan d'illumination et d'observation, c'est à dire du plan d'observation confocale des tissus est obtenue soit en déplaçant l'interface de sortie du guide d'images à fibres optiques 300, c'est à dire l'interface situé du côté des tissus, par rapport au bloc optique 400 qui dans ce cas reste fixe et est situé devant le tissu, soit en déplaçant l'ensemble des lentilles L1, L2, L3 selon l'état de l'art afin de changer le plan focal de mise au point dans le corps tout en maintenant le champ d'exploration constant.

Dans ce deuxième cas, une exploration tomographique peut être effectuée sans modification, d'une part du champ de tissus exploré, et sans modification d'autre part de l'ouverture numérique. De tels déplacements indépendants du diamètre de l'interface de sortie du guide d'images, c'est à dire de l'élément filtrant comme il sera décrit ci-après, permettent de choisir indépendamment la position du corps observé et l'épaisseur de la coupe de tissus observée.

La partie optique située à l'extrémité du guide d'images du côté des tissus observés réalise donc une illumination de micro-surfaces de tissus. Le flux réfléchi par cette petite surface de tissu éclairée durant un bref instant emprunte le chemin inverse de celui de l'illumination. Ainsi, les rayons réfléchis et rétrodiffusés par la micro surface éclairée traversent successivement la lentille L1, la lame LM1, la lentille L2, la lentille L3, la fibre optique du groupe de fibres qui est éclairée pendant ce bref instant, puis ce faisceau d'observation repasse par le dispositif de balayage mécano-optique 200, au niveau duquel il est stabilisé.

Le faisceau d'observation arrive alors sur une lame LM2 dichroïque disposée à 45° sur le trajet du faisceau mélangé d'illumination et d'observation entre le déviateur 200 et la source 100. Cette lame LM2 est choisie pour laisser passer le flux d'illumination allant de la source 100 vers les fibres optiques et pour réfléchir à 90° les rayons d'observation provenant des fibres optiques.

La lame LM2 situé côté observateur par rapport aux fibres est donc destinée à assurer la séparation entre les faisceaux d'illumination et la lumière rétrodiffusée par les tissus étudiés. Selon une disposition avantageuse de l'invention, la lame LM2 est une lame séparatrice dont les caractéristiques varient en fonction de la longueur d'onde. De même que pour la lame LM1 précédemment décrite, la lame LM2 peut être remplacée par un prisme ou un cube apte à séparer les rayons ayant des sens de propagation opposés.

Le dispositif de séparation des voies d'illumination et de détection le plus simple est constitué par cette lame LM2 favorisant le flux issu des tissus. Tout autre dispositif susceptible de séparer le flux d'illumination du flux rétro diffusé par le tissu pourra être utilisé pour remplacer cette lame LM2. Ainsi, des filtres interférentiels peuvent être interposés sur la voie de détection dans le cas d'une image de fluorescence. On peut également utiliser une séparation à l'aide d'un dispositif séparateur de polarisation en utilisant une source d'illumination polarisée. Ce dispositif sera intéressant pour étudier l'autofluorescence des tissus ou la fluorescence provenant d'un colorant circulant et/ou fixé dans les tissus excités par une lumière d'illumination dont la longueur d'ondes est convenablement choisie.

Le flux d'observation est alors focalisé par une lentille L5 sur un filtre spatial sélectif 600. Derrière le filtre spatial sélectif 600, des éléments photosensibles 700 reçoivent ce flux après passage par le filtre spatial 600 qui est ici un trou de filtration spatiale. Chaque micro-surface tissulaire illuminée est ainsi conjuguée avec l'élément de filtration spatiale, par l'intermédiaire du système optique formé par les lentilles L1, L2, L3, L4 et L5. Chaque micro-surface de tissu éclairée est ainsi imagée.

Le dispositif de filtration 600 est ici constitué d'un trou de filtration spatiale à la limite de la diffraction ou légèrement supérieure à celle-ci. Il est destiné à éliminer les lumières parasites issues des fibres latérales à la fibre d'illumination. Le diamètre de ce trou de filtration spatiale est fixé par les caractéristiques de l'optique et le diamètre de fibres du guide d'images. Le diamètre est déterminé de façon à ce que la lumière issue de la fibre d'illumination et de réception soit la seule analysée. Cette pupille de filtration est, comme on l'a décrit précédemment, disposée dans le plan conjugué ou confocal des tissus.

Ainsi, le dispositif de filtration spatiale est situé dans un plan image conjugué à l'extrémité du guide de fibres optiques adjacente au séparateur LM2. Ce dispositif de filtration participe donc à l'élimination des reflets parasites et il est conjugué de l'extrémité de la fibre qui est située du côté du détecteur. Ce filtre permet de sélectionner uniquement la fibre éclairée à un instant considéré. Ce filtre est disposé dans le plan conjugué des tissus, devant le détecteur destiné à les imager.

Le capteur 700 est ici un capteur photosensible de type photomultiplicateur conjugué optiquement du point d'observation des tissus. Ce capteur peut également être une photo diode à avalanches ou tout autre moyen de détection permettant une mesure de flux lumineux. Ce bloc de réception opto-électronique peut être également constitué d'un ou plusieurs capteurs, amplifiés, ou refroidis, qui permettent de fournir une image électronique.

Un éventuel dispositif de filtration chromatique peut permettre de disposer plusieurs photodétecteurs qui réalisent en parallèle les images des tissus formés par les diverses longueurs d'ondes d'illumination. Pour chaque point du photodétecteur le nombre de niveaux dénombrables dépend du rapport entre le flux maximum parvenant au détecteur et le flux parasite. Entre le filtre spatial 600 et le photodétecteur 700 est disposée une lame à 45° LM3 déviant vers un second photodétecteur 900 le flux rétrodiffusé par le corps et capté par la lame LM1 et laissant les autres rayons issus de la vue axiale se propager jusqu'au photodétecteur axial 700.

En arrière du photodétecteur 700 est disposé un bloc électronique 800 permettant de traiter le signal recueilli et de délivrer un signal électrique susceptible de commander un émetteur de visualisation et/ou un magnétoscope et/ou d'être traité dans un micro-ordinateur. Cette structure 10 permet de donner une image dimensionnelle d'une coupe optique de tissus.

Par numérisation et mémorisation des coupes à différentes profondeurs, on reconstitue une image tridimensionnelle des tissus ou de la surface industrielle étudiée.

Ainsi, le photodétecteur 700 et le dispositif 800 ainsi qu'un moniteur de visualisation constituent un ensemble de présentation d'une image de l'intérieur du corps dans lequel le photodétecteur 700 constitue donc un bloc de réception opto-électronique permettant de transformer un signal optique en un signal électrique.

On comprend donc qu'avec le dispositif qui vient d'être décrit, la surface éclairée correspond à un point de l'image observée. Le déplacement de cette micro-surface d'illumination est assuré par un adressage optique successif des fibres optiques du groupe de fibres 300. C'est la juxtaposition de ces différentes mesures de flux qui permet de reconstituer l'image.

Pour cela, le dispositif comporte des moyens aptes à enregistrer des informations lumineuses provenant des parties éclairées du corps au cours du balayage et aptes à enregistrer avec ces informations lumineuses des informations sur la position de la partie éclairée dans la zone balayée, pour reconstituer une image à partir de ces deux groupes d'informations.

Le dispositif qui vient d'être décrit comporte un filtre spatial sélectif 600. Toutefois, au sein d'un tel dispositif, un élément principal de filtration est fourni par une filtration confocale assurée par l'extrémité tissulaire du guide d'images à fibres optiques 300.

En effet, une pupille de filtration est constituée par l'extrémité de chaque fibre du guide d'images 300 qui est situé du côté des tissus à étudier. Une filtration confocale est donc assurée par l'extrémité de la fibre du guide qui est illuminé à l'instant t. Un tel dispositif peut être appelé « dispositif de filtration confocale ou conjuguée ». Le diamètre réduit de cette pupille de filtration lié au diamètre de la fibre, permet d'éliminer les reflets parasites issus des fibres adjacentes.

Pour améliorer encore le filtrage et ainsi la détection de très faibles flux (de l'ordre du nW) issu des tissus, on s'affranchit des réflections issues des deux extrémités du guide d'images à fibre optique.

Pour ce faire, à l'extrémité située du côté tissulaire, les fibres sont clivées ou polies de façon à présenter avec le plan perpendiculaire à l'axe de la fibre un angle suffisant pour permettre de s'affranchir de la réflection qui est alors en mode dit « à fuites ».

Plus généralement on veille à réaliser l'extrémité des fibres côté corps selon un plan d'extrémité qui ne soit pas perpendiculaire à un axe de sortie de la fibre à cette extrémité. Un tel plan peut être réalisé par clivage ou par polissage.

En outre, à l'extrémité située vers l'injection du flux d'illumination, une stimulation par train d'impulsions lumineuses est utilisée pour la stimulation lumineuse de la source 100.

L'invention décrite ici utilise un dispositif de balayage opto-mécanique permettant d'adresser successivement les différentes fibres constituant le guide d'images de l'endoscope. Cet adressage successif des différentes fibres autorise une filtration spatiale confocale des images obtenues, procurant une résolution en profondeur de type microscopique confocal.

Le dispositif étant confocal, en d'autres termes le point illuminé étant le conjugué optique de la source et d'un détecteur, il récupère les rayons réfléchis et rétrodiffusés par le point momentanément illuminé en discriminant ces rayons des autres rayons provenant de la diffusion dans les tissus environnant ce point.

L'ensemble formé par le module 400, le guide d'images 300 et le déviateur 200 présente donc une entrée de réception optique formée par la lentille L1, et est adapté pour ne transmettre jusqu'aux moyens de présentation d'image que les rayons de retour arrivant sur la lentille directement depuis le point de concentration de lumière.

Le module 400 dirige donc les rayons lumineux provenant de la partie illuminée de la zone sur l'extrémité coté corps de la fibre d'éclairement de sorte que la fibre de retour et la fibre d'éclairement sont une même fibre.

Cette filtration est obtenue ici par le fait que les moyens optiques conjuguent optiquement le point illuminé à un capteur photométrique, mais on peut dans le cadre de l'invention utiliser un autre moyen pour réaliser une tel filtrage.

En permettant de plus de choisir le plan d'observation dans un milieu diffusant, c'est à dire le plan dont on extrait les signaux, il permet de réaliser un image en trois dimensions d'un partie du corps à observer.

Dans le mode de réalisation décrit ici, une commande de l'élément émissif 100 est adoptée. Selon une variante, l'invention prévoit l'interposition d'une porte optique de type modulaire acousto-optique ou électro-optique ou une commande directe des éléments d'illumination et de détection.

Selon une variante, les moyens d'illumination comprennent en effet une source fournissant des trains d'impulsions lumineuses, et les moyens de présentation d'images comprennent des moyens de détection des rayons provenant du corps, aptes à sélectionner temporellement ces rayons de manière temporellement adaptée à l'émission des impulsions lumineuses.

Grâce à une telle stimulation par train d'impulsions lumineuses, le flux réfléchi est analysé de façon synchrone en tenant compte du temps de transit le long de la voie optique, c'est à dire pour le trajet aller-retour dans les blocs optiques, guides d'images et tissus.

Ainsi, les signaux réfléchis arrivant au photodétecteur 700 sensiblement simultanément à l'émission d'un éclairement par la source 100 sont analysés en rapport avec cette émission.

La commande de délai entre l'émission et la réception est assurée par un dispositif électronique de commande. Les délais de la durée des impulsions d'illumination sont correctement calculés et ajustés pour chaque longueur de guides d'images et de chaque type de dispositif optique. Des flux résiduels éventuels issus des portes optiques imparfaites sont éliminés par un dispositif de polarisation entre l'émission et la réception.

Ainsi, dans la variante précédemment citée, un dispositif de porte optique et/ou électronique commandé par le dispositif de commande 800 permet de réaliser la détection synchrone du flux réfléchi. Ainsi des rayons réfléchis parasites ne sont pas traités.

Le dispositif de commande 800 comporte préférentiellement un bloc permettant de générer la séquence de tous les événements et de traiter tous les signaux issus de capteurs et éventuellement de différents éléments de modulation du flux d'illumination ou émanant des éléments de commande de l'amplification de réception.

Ce dispositif horloge commande les portes de détection synchrones de façon à tenir compte du balayage des différentes fibres et du temps de transit optique entre les sources et les analyseurs de flux.

Plus précisément, dans la présente variante préférentielle, le module de commande 800 positionne, comme décrit précédemment, le déviateur 200 selon des positions successives correspondant chacune à une fibre différente du groupe de fibres 300.

Pour chaque position du déviateur, il dévie le faisceau d'illumination sur la lentille L4 pour que celui ci ressorte de cette lentille face à une fibre choisie et dans l'alignement de cette fibre.

Ce faisceau, après avoir parcouru la fibre, rencontre le corps observé, et, après avoir été retrodiffusé par les tissus et avoir parcouru la fibre dans le sens opposé, ressort de cette même fibre selon le même angle que le faisceau d'illumination, parcourt le même trajet que le faisceau d'illumination à travers la lentille L4 et le déviateur 200, dirigé ainsi sur un orifice de filtration 600.

Pour chacune de ses positions, le déviateur 200 dirige le faisceau d'illumination sur une fibre choisie puis recentre et réaligne le faisceau de retour, sans changer de position.

Selon une disposition préférée, on utilise une fibre optique pour chaque pixel de représentation de l'image sur un écran vidéo, et le déviateur adopte un balayage synchronisé avec un balayage effectuée par des moyens de balayage de l'écran vidéo.

En d'autres termes, le déviateur 200 reste sur une fibre pendant un intervalle de temps dicté par la durée d'un pixel d'écran vidéo.

On divise avantageusement cet intervalle de temps en deux demi-intervalles distincts sans plage intermédiaire, qui correspondent respectivement à une première phase d'illumination du corps par la source et une deuxième phase de réception de la lumière rétrodiffusée du corps vers le photodétecteur 700.

Pour mettre en oeuvre ces deux phases sans croisement gênant de la lumière et sans laisser d'intervalle de non-exploitation de la fibre entre les deux phases, on choisit un longueur de fibre telle que la durée d'un aller-retour de la lumière soit égale à la moitié de la durée de positionnement du déviateur face à la fibre.

Ainsi, la lumière retro-diffusée, qui commence à arriver sur le déviateur 200 après avoir effectué un aller-retour, commence à arriver à la fin de la première moitié de l'intervalle de positionnement du déviateur 200, instant où l'émission du faisceau d'illumination est arrêtée.

L'instant situé au demi-intervalle de positionnement du déviateur 200 correspond donc simultanément à la disparition du faisceau d'illumination et à l'apparition du faisceau de retour au niveau du déviateur.

On évite ainsi un croisement de la lumière à l'extrémité de la fibre qui est adjacente au déviateur, ce qui engendrerait une forte lumière parasite, tout en profitant de l'ensemble de la durée de positionnement du déviateur 200, puisque les parties voisines du déviateur sont à tout moment parcourues par un faisceau exploité, en direction d'aller puis en direction de retour.

Une telle longueur de fibre est, pour les durées de pixel des écrans vidéo actuels, de l'ordre de 10 mètres.

On utilise donc des fibres dont la longueur dépasse largement les longueurs dictées jusqu'à présent par les dimensions du corps à observer. On enroule les fibres pour diminuer leur encombrement.

On prévoit selon une variante de l'invention d'adopter un dispositif d'hétérodynage optique, un dispositif interférométrique ou un dispositif spectrométrique.

L'ensemble des dispositifs décrits ci-dessus permet de réaliser des flux réfléchis qui sont de l'ordre de 1/10⁶ du flux parasite issu des différentes surfaces de l'architecture optique.

Dans une disposition particulière de l'appareil et lorsque les flux réfléchis sont inférieurs au nW, une branche optique à fibres munie d'un miroir à distance réglable permet de réaliser une amplification optique hétérodyne basée sur les principes de l'interférométrie.

Le dispositif décrit ci-dessus permet le repérage des tissus sous un angle de 20° X 20° et la visualisation de coupes optiques microscopiques confocales sous un angle de 10°. Parallèlement à cette coupe optique microscopique confocale, l'explorateur a une vue axiale large champ lui permettant de se repérer.

L'exploration large champ se fait au moyen de lentilles L3, L1 et L5 suivant les règles de l'art pour fournir une image de l'ordre de 2 à 3 cm dans le plan image de la lentille L5. C'est la lentille L5 qui focalise le flux sur le détecteur. Cette exploration représente de l'ordre de 40° de champ. L'image totale formée est renouvelée 25 fois/seconde pour 100X100 points résolus. On utilise donc ici une technique dite du point volant appelée « flying spot ».

Dans une image tissulaire de 100x100 points couvrant un champ de 400 µm, la résolution est de 4 µm.

Le fait d'utiliser la même fibre optique pour l'illumination et l'observation présente de nombreux avantages.

Ainsi, le fait, contrairement aux dispositifs connus, de ne pas utiliser la règle de séparation des pupilles d'illumination et d'observation, permet d'utiliser une pupille optique permettant une ouverture numérique importante. Un tel montage autorise donc une très bonne résolution d'image, celle-ci étant conditionnée uniquement par la qualité du guide utilisé. Cette qualité de guide dépend de ses caractéristiques propres et notamment des diamètres des fibres utilisées.

L'utilisation d'une même fibre optique pour réaliser l'illumination des tissus et l'analyse du flux réfléchi permet la conjugaison entre le point source et le point de filtration spatiale du flux réfléchi, qui est toujours assuré sans nécessiter de réglage puisque le point source, ou point illuminé ainsi que le point de filtration spatiale, ou point observé sont dépendants du même élément optique, c'est à dire notamment des mêmes lentilles de sortie mais surtout de la même fibre optique.

D'une manière plus générale, l'utilisation de fibres optiques permet de disposer à distance le dispositif d'illumination 100 et de balayage 200 et le dispositif de détection 600, 700, 800 tout en conservant un mode d'analyse point par point de type point volant ou « flying spot ».

Le fait d'utiliser des fibres optiques permet d'obtenir l'indépendance entre le champ observé, l'ouverture numérique et la position axiale en Z du plan d'observation, c'est à dire la position axiale en profondeur.

Le dispositif de modulation du faisceau d'illumination, le dispositif de réflection et de balayage des fibres permettant une détection synchrone peuvent également être situés à distance. L'électronique de commande et les différents composants optiques encombrants sont également éloignés des tissus observés. De plus, la partie située devant le tissu à observer est de volume très réduit (quelques mm³ à quelques cm³).

Un intérêt d'utilisation d'un guide d'images à fibres optiques est donc de pouvoir déporter les sources, les déflecteurs effectuant le balayage bidimensionnel du champ d'illumination et d'observation. Cette disposition conditionne la miniaturisation et l'utilisation des dispositifs situés devant les tissus dans toutes les positions.

Le fait de conserver le même système de guides d'images, quelque soit le champ à observer permet de conserver les caractéristiques de mise au point du plan d'observation.

Une des caractéristiques de l'invention décrite dans ce dispositif consiste à observer des images simultanément dans l'axe avec un grand champ et latéralement avec un petit champ et une grande résolution. Tandis qu'une des longueurs d'onde est utilisée pour l'observation axiale, l'autre est utilisée pour la coupe microscopique confocale. Il est donc possible de visualiser simultanément l'image axiale et la coupe optique confocale sur deux écrans différents.

Deux dispositifs optiques sont donc combinés afin de permettre une image axiale macroscopique et une image latérale microscopique.

L'un des intérêts de l'invention est l'emploi de fibres optiques dont le choix de matériaux et de leur usinage, contribue à définir l'ouverture numérique, alors que le champ d'observation est fixé par la focale des lentilles situées devant les tissus.

Le changement de l'ouverture numérique de la fibre et/ou du diamètre de limitation du faisceau d'illumination permet de modifier l'ouverture du système optique et donc de changer la résolution spatiale. Par ailleurs, le changement de diamètre des fibres permet de modifier l'épaisseur de la coupe optique sans réserve de conserver le même grossissement.

Ainsi, on vient de décrire un dispositif permettant d'obtenir des performances au moins identiques aux dispositifs conventionnels avec un encombrement beaucoup plus réduit, car le même contingent du guide d'images est utilisé pour l'illumination et l'analyse de la réflection tissulaire.

Bien entendu, l'invention ne se limite pas au seul exemple qui vient d'être décrit.

Selon une variante de l'invention de type miniaturisée, le trajet optique nécessaire est obtenu par repliement du faisceau. Les pertes induites sont alors compensées par une augmentation de la puissance de la source en se limitant aux valeurs des normes AFNOR et ANSI.

De même, l'exemple de réalisation de l'invention précédemment décrite a été présenté comme destiné à observer un tissu biologique. Grâce à un dispositif selon l'invention, toute autre surface peut être étudiée, notamment en milieu industriel, en temps réel. L'invention concerne donc à la fois l'exploration de dispositifs manufacturés et celle d'organismes vivants, ou encore l'exploration de l'intérieur de corps minéraux.

L'invention ne se limite pas à des applications en médecine et en biologie mais s'adresse également à tout type d'étude, notamment en milieu industriel, par exemple le contrôle de canalisations ou de turbines en milieu polluant et/ou inaccessible à l'observation directe, et plus généralement hostile à l'homme, et plus généralement l'observation de toute autre surface.

Grâce à l'invention, on place avantageusement l'ensemble des éléments électroniques, mécaniques et plus généralement l'ensemble des éléments attirés ou susceptibles d'être attirés par un champ magnétique à l'extrémité du guide qui est opposée au patient.

Les matériaux destinés à être en contact avec les tissus sont choisis de manière à ne pas perturber les champs magnétiques. La partie voisine du patient n'étant pas attirée par un champ magnétique, elle peut être placée sous un dispositif d'observation par imagerie par résonance magnétique IRM afin de pouvoir effectuer des observations conjointes et simultanées, endoscopiques et IRM.

Ce dispositif permet une observation à distance des tissus, la longueur de plusieurs mètres du guide d'images permettant de faire une imagerie endoscopique microscopique confocale, tout en effectuant conjointement une exploration par résonance magnétique nucléaire.

Dans le mode de réalisation préféré de l'invention, un opérateur accède aux tissus avec un autre instrument optique tel qu'un laser de photo-coagulation en empruntant le guide d'images 300.

Le bloc source de lumière 100 est pour cela prévu pour fournir sur commande un faisceau particulièrement énergétique, qui produit sur les tissus (plus généralement la matière constitutive du corps observé) un traitement ou une transformation, ici une photo-coagulation. Ce traitement peut être également une photo-destruction, ou plus généralement un traitement thérapeutique photonique dans le cas de l'utilisation du dispositif sur un corps vivant.

Le module de commande électronique 800 inclut un module de sélection de fibres prévu pour commander le bloc source 100 en fonction du positionnement du déviateur 200.

L'utilisateur indique sur l'écran de visualisation une fenêtre ou zone, comprise dans l'image représentée à l'écran, sur laquelle il souhaite effectuer le traitement.

L'image représentée correspondant à l'ensemble des fibres du groupe de fibres 300, la zone indiquée par l'utilisateur correspond, à un sous-groupe de fibres que le module de sélection déduit automatiquement de l'indication donnée par l'utilisateur.

Les références des fibres sélectionnées sont prises en compte par l'unité de commande 800, qui commande le bloc source 100 en fonction du positionnement du déviateur 200 au cours du balayage ordinaire, de telle sorte que le bloc 100 fournit un faisceau particulièrement énergétique, ici un faisceau laser thérapeutique lorsque le déviateur 200 est dirigé sur une des fibres sélectionnées, et de telle sorte que le bloc 100 ne fournit qu'un faisceau d'éclairement ordinaire lorsque le déviateur est dirigé vers une fibre non sélectionnée.

Selon le présent mode de réalisation, le bloc source 100 comprend deux sources 110 et 115, l'une 110 pour le traitement (laser par exemple) et l'autre 115 pour l'éclairement simple, dont le module 800 active l'une ou l'autre selon la fibre illuminée.

Selon une variante, on utilise une seule source apte sur commande, à émettre en fonction de la fibre illuminée un faisceau plus ou moins puissant, à effet de traitement ou non selon cette puissance.

On adopte également dans un tel dispositif un module de traitement des signaux électriques fournis par le photodétecteur 700 qui est commandé par le module 800 de manière synchronisée au positionnement du déviateur 200 pour appliquer un traitement particulier aux faisceaux reçus des fibres sélectionnées et compenser ainsi l'intensité plus forte reçue des parties en cours de traitement.

La lumière de traitement est donc utilisée également comme lumière d'éclairement, qui est analysée après rétro-diffusion.

On utilise donc à chaque fois ici une même fibre pour véhiculer simultanément la lumière de traitement, la lumière d'éclairement et la lumière d'observation de retour.

Selon une variante on peut utiliser une fibre différente pour véhiculer le faisceau d'observation et le faisceau de traitement.

On réalise ici par une même fibre à la fois une observation et un traitement qui peut être thérapeutique.

On véhicule également, selon une autre variante, sur une même fibre, un signal utilisé par un dispositif d'analyse spectrométrique, par exemple dans un but diagnostique. Un dispositif rassemblant ces trois fonctions véhicule alors sur un même guide d'image des signaux utilisés dans trois buts, celui d'observer, celui de diagnostiquer et celui de traiter.

## Revendications

1. Dispositif pour observer l'intérieur d'un corps, comprenant :
- un groupe de fibres (300) réunies côte à côte, destiné à parcourir une partie intérieure du corps, comportant une extrémité extérieure ou proximale et une extrémité à l'intérieur du corps ou extrémité distale, ledit groupe de fibres formant un guide d'image dont les extrémités sont ordonnées dans le même ordre aux deux extrémités respectivement intérieure et extérieure dudit groupe de fibres,
- des moyens (110, 115) du côté proximal pour émettre un faisceau d'illumination prévu pour être acheminé via le groupe de fibres depuis l'extrémité extérieure ou proximale vers une zone du corps à observer,
- des moyens (200) du côté proximal pour balayer l'extrémité extérieure ou proximale du groupe de fibres avec ce faisceau d'illumination ces moyens de balayage étant commandés pour imprimer des déviations angulaires successives au faisceau d'illumination de manière à adresser successivement l'extrémité extérieure ou proximale de chaque fibre ou ensemble de fibres au sein du groupe de fibres formant guide d'image, de façon à former à l'extrémité intérieure ou distale de ladite fibre une source lumineuse secondaire à un instant donné, ladite source lumineuse secondaire se déplaçant successivement d'une fibre à l'autre ,
- des moyens du côté distal pour prélever un faisceau rétrodiffusé provenant de la zone illuminée et l'acheminer via le groupe de fibres vers des moyens du côté proximal pour recevoir ledit faisceau rétrodiffusé,
- des moyens pour produire à partir du faisceau rétrodiffusé reçu une image de la zone illuminée, et
- des moyens pour commander les moyens d'émission lumineuse et les moyens de balayage, et coopérant avec les moyens de production d'image,
**caractérisé en ce qu'**il comprend en outre des moyens (L1, L2, L3, L4, L5) pour former un plan image conjugué de la zone illuminée à l'intérieur du corps, ainsi qu'un orifice de filtration (600) pour filtrer spatialement le faisceau rétrodiffusé reçu, l'orifice étant situé en amont des moyens de production d'image dans le plan image conjugué de la zone illuminée à l'intérieur du corps et agencé de sorte que seule la lumière rétrodiffusée issue de

2. Dispositif selon la revendication 1, **caractérisé en ce que** chaque fibre illuminée est également utilisée pour véhiculer un faisceau de retour provenant de la zone du corps illuminée par elle, et **en ce que** les moyens de balayage comprennent des moyens pour imprimer des déviations angulaires (200) successives du faisceau d'illumination arrangés pour faire parcourir à un faisceau de retour sortant de cette même fibre le même chemin optique que celui du faisceau d'illumination entrant dans cette fibre.

3. Dispositif selon la revendication 2, **caractérisé en ce que** pour chaque déviation angulaire successive, une fibre (300) correspondant à cette déviation présente une longueur telle que la durée de propagation aller et retour de la lumière dans la fibre (300) est égale à la moitié de la durée pendant laquelle le moyen d'illumination conserve ladite déviation angulaire, et les moyens d'illumination (110, 115, 200) illuminent la fibre pendant la première moitié de l'intervalle de temps où le moyen d'illumination conserve ladite déviation angulaire.

4. Dispositif selon l'une quelconque des revendications précédentes, **caractérisé en ce qu'**il comporte deux dispositifs de présentation d'image l'un adapté pour présenter une vue d'une zone générale et l'autre une vue microscopique d'une zone plus restreinte et **en ce qu'**il comporte des moyens (LM1, LM2) pour présenter simultanément les deux vues sur les deux dispositifs de présentation d'image .

5. Dispositif selon l'une quelconque des revendications précédentes, **caractérisé en ce que** les moyens d'illumination sont prévus pour illuminer des fibres (300) avec une lumière produisant une transformation de la matière du corps.

6. Dispositif selon la revendication 5, **caractérisé en ce que** les moyens de présentation de l'image (600, 700, 800) comprennent des moyens permettant à un utilisateur de sélectionner sur l'image présentée une zone à traiter, ces moyens étant prévus pour convertir l'indication de cette zone en des références de fibres et pour commander aux moyens d'illumination (110, 115, 200) d'illuminer les fibres correspondant à ces références avec la lumière produisant ladite transformation.

7. Dispositif selon l'une quelconque des revendications précédentes, **caractérisé en ce qu'**il comporte des moyens (110, 115, 200, 300, 400) pour produire dans la zone du corps balayée un point de concentration des rayons lumineux d'éclairement.

8. Dispositif selon la revendication 7, **caractérisé en ce qu'**il comprend en outre des moyens de réglage de position en profondeur du point de concentration des rayons lumineux ainsi que des moyens de mémorisation des images reçues pour différents réglages de position en profondeur afin de fournir une image tridimensionnelle de la partie du corps observée.

9. Dispositif selon les revendications 7 et 8, **caractérisé en ce que** les moyens de concentration (400) des rayons sont placés à l'extrémité côté corps du guide d'image et **en ce que** les moyens de réglage de la position en profondeur du point de concentration sont constitués par des moyens de réglage de l'éloignement des moyens de concentration par rapport à l'extrémité côté corps du guide d'image.

10. Dispositif salon l'une des revendications 7 à 9, **caractérisé en ce qu'**il comporte des moyens (L1, L2, L3, 300, 200) ayant une entrée de réception optique (L1), ces moyens étant adaptés pour ne transmettre jusqu' aux moyens de présentation d'image (600, 700, 800) que les signaux obtenus à partir de rayons lumineux de retour arrivant directement du point de concentration dans l'entrée de réception optique (L1).

11. Dispositif selon la revendication 10, **caractérisé en ce que** les moyens de concentration des rayons lumineux et les moyens à entrée de réception (400) sont formés par un même module (400), le faisceau d'éclairement et le faisceau de retour ayant même trajet au sein de ce module (400).

12. Dispositif selon l'une quelconque des revendications précédentes, **caractérisé en ce qu'**il comprend en outre des moyens (800) pour enregistrer des informations lumineuses provenant des parties éclairées du corps au cours du balayage et aptes à enregistrer avec ces informations lumineuses des informations sur la position de la partie éclairée dans la zone balayée, pour reconstituer une image à partir de ces deux groupes d'informations.

13. Dispositif selon l'une des revendications précédentes, **caractérisé en ce que** l'extrémité distale ou côté corps d'au moins une fibre optique forme un plan non perpendiculaire à l'axe de sortie de la fibre.

14. Dispositif selon l'une des revendications précédentes, **caractérisé en ce qu'**il est adapté pour l'observation de l'intérieur du corps d'un être vivant.

15. Dispositif selon l'une des revendications précédentes, **caractérisé en ce qu'**il est adapté pour l'observation de l'intérieur d'un dispositif manufacturé.

## Patentansprüche

1. Vorrichtung zum Beobachten des Inneren eines Körpers, umfassend:
- eine Gruppe von nebeinander zusammengefügten Fasern (300), die dazu bestimmt ist, einen inneren Bereich des Körpers zu durchqueren, und ein äußeres oder proximales Ende und ein Ende im Inneren des Körpers oder distales Ende aufweist, wobei die Faserngruppe einen Bildleiter bildet, dessen Enden an den beiden Enden, dem inneren Ende bzw. dem äußeren Ende, dieser Faserngruppe in der gleichen Ordnung angeordnet sind,
- proximalseitige Mittel (110,115) zum Aussenden eines Beleuchtungsbündels, das dafür vorgesehen ist, über die Faserngruppe von dem äußeren oder proximalen Ende zu einer Zone des zu beobachtenden Körpers befördert zu werden,
- proximalseitige Mittel (200) zum Abtasten des äußeren oder proximalen Endes der Faserngruppe mit diesem Beleuchtungsbündel, wobei diese Abtastmittel so gesteuert werden, daß sie dem Beleuchtungsbündel aufeinanderfolgende Winkelablenkungen verleihen, so daß das äußere oder proximale Ende jeder Faser oder jedes Fasernsatzes innerhalb der den Bildleiter bildenden Faserngruppe nacheinander adressiert wird, so daß am inneren oder distalen Ende dieser Faser zu einem gegebenen Zeitpunkt eine sekundäre Lichtquelle gebildet wird, die sich nacheinander von einer Faser zur anderen bewegt,
- distalseitige Mittel zum Entnehmen eines von der beleuchteten Zone kommenden zurückgestreuten Bündels und zu seiner Beförderung über die Faserngruppe zu proximalseitigen Mitteln zum Empfangen dieses zurückgestreuten Bündels,
- Mittel zum Erzeugen eines Bildes der beleuchteten Zone aus dem empfangenen zurückgestreuten Bündel und
- mit den Bilderzeugungsmitteln zusammenarbeitende Mittel zum Steuern der Lichtaussendungsmittel und der Abtastmittel,
**dadurch gekennzeichnet, daß** sie ferner Mittel (L1,L2,L3,L4,L5) zum Bilden einer zur beleuchteten Zone im Inneren des Körpers konjugierten Bildebene aufweist, sowie eine Filteröffnung (600) zur räumlichen Filterung des empfangenen zurückgestreuten Bündels, wobei diese Öffnung stromauf der Bilderzeugungsmittel in der zur beleuchteten Zone im Inneren des Körpers konjugierten Bildebene gelegen ist und so ausgebildet ist, daß nur das zurückgestreute Licht, das von dem äußeren oder proximalen Ende der zu einem gegebenen Zeitpunkt adressierten Faser kommt, durch die Bilderzeugungsmittel verarbeitet wird.

2. Vorrichtung nach Anspruch 1, **dadurch gekennzeichnet, daß** jede beleuchtete Faser auch zur Beförderung eines zurückkehrenden Bündels, das von der durch diese Faser beleuchteten Zone des Körpers kommt, verwendet wird und daß die Abtastmittel Mittel (200) umfassen, die dem Beleuchtungsbündel aufeinanderfolgende Winkelablenkungen verleihen und ausgelegt sind, um ein aus derselben Faser austretendes zurückkehrendes Bündel denselben optischen Weg wie das in diese Faser eintretende Beleuchtungsbündel durchlaufen zu lassen.

3. Vorrichtung nach Anspruch 2, **dadurch gekennzeichnet, daß** bei jeder aufeinanderfolgenden Winkelablenkung eine dieser Ablenkung entsprechende Faser (300) eine solche Länge besitzt, daß die Ausbreitungsdauer des Lichts in der Faser (300) hin und zurück gleich der Hälfte der Zeit ist, während der das Beleuchtungsmittel diese Winkelablenkung beibehält, und die Beleuchtungsmittel (110,115,200) die Faser während der ersten Hälfte des Zeitraums beleuchten, in dem das Beleuchtungsmittel diese Winkelabweichung beibehält.

4. Vorrichtung nach einem der vorhergehenden Ansprüche, **dadurch gekennzeichnet, daß** sie zwei Bilddarstellungsvorrichtungen aufweist, deren eine dafür ausgelegt ist, eine Ansicht einer allgemeinen Zone darzustellen, und deren andere dafür ausgelegt ist, eine mikroskopische Ansicht einer engeren Zone darzustellen, und daß sie Mittel (LM1,LM2) aufweist, um die beiden Ansichten auf den beiden Bilddarstellungsvorrichtungen gleichzeitig darzustellen.

5. Vorrichtung nach einem der vorhergehenden Ansprüche, **dadurch gekennzeichnet, daß** die Beleuchtungsmittel vorgesehen sind, um Fasern (300) mit einem eine Umwandlung der Substanz des Körpers erzeugenden Licht zu beleuchten.

6. vorrichtung nach Anspruch 5, **dadurch gekennzeichnet, daß** die Bilddarstellungsmittel (600,700,800) Mittel umfassen, die einem Benutzer gestatten, auf dem dargestellten Bild eine Behandlungszone auszuwählen, und die vorgesehen sind, um die Anzeige dieser Zone in Fasernbezugsgrößen zu überführen und um die Beleuchtungsmittel (110,115,200) so zu steuern, daß sie die diesen Bezugsgrößen entsprechenden Fasern mit dem diese Umwandlung erzeugenden Licht beleuchten.

7. Vorrichtung nach einem der vorhergehenden Ansprüche, **dadurch gekennzeichnet, daß** sie Mittel (110,115,200,300,400) zum Erzeugen eines Konzentrationspunkts der Beleuchtungslichtstrahlen in der Zone des abgetasteten Körpers aufweist.

8. Vorrichtung nach Anspruch 7, **dadurch gekennzeichnet, daß** sie außerdem Mittel zur Einstellung der Tiefenstellung des Konzentrationspunkts der Lichtstrahlen sowie Mittel zur Speicherung der Bilder aufweist, die bei verschiedenen Tiefeneinstellungen empfangen werden, um ein dreidimensionales Bild des beobachteten Körperbereichs zu liefern.

9. Vorrichtung nach den Ansprüchen 7 und 8, **dadurch gekennzeichnet, daß** die Mittel (400) zur Konzentration der Strahlen an dem körperseitigen Ende des Bildleiters angeordnet sind und daß die Mittel zur Einstellung der Tiefenstellung des Konzentrationspunktes aus Mitteln zur Einstellung der Entfernung der Konzentrationsmittel von dem körperseitigen Ende des Bildleiters bestehen.

10. Vorrichtung nach einem der Ansprüche 7 bis 9, **dadurch gekennzeichnet, daß** sie Mittel (L1,2,L3,300,200) umfasst, die einen optischen Empfangseintritt (L1) aufweisen und die dafür ausgelegt sind, bis zu den Bilddarstellungsmitteln (600,700,800) nur die Signale zu übertragen, die aus zurückkehrenden Lichtstrahlen erhalten werden, die im optischen Empfangseintritt (L1) direkt vom Konzentrationspunkt ankommen.

11. Vorrichtung nach Anspruch 10, **dadurch gekennzeichnet, daß** die Mittel zur Konzentration der Lichtstrahlen und die Mittel mit Empfangseintritt (400) von einem gemeinsamen Modul (400) gebildet sind, wobei das Beleuchtungsbündel und das zurückkehrende Bündel im Inneren dieses Moduls (400) denselben Weg durchlaufen.

12. Vorrichtung nach einem der vorhergehenden Ansprüche, **dadurch gekennzeichnet, daß** sie außerdem Mittel (800) zum Aufzeichnen der Lichtinformationen aufweist, die von den während der Abtastung beleuchteten Körperbereichen kommen, wobei diese Mittel dafür ausgelegt sind, mit diesen Lichtinformationen Informationen über die Lage des beleuchteten Bereichs in der abgetasteten Zone aufzuzeichnen, um aus diesen beiden Gruppen von Informationen ein Bild zu rekonstituieren.

13. Vorrichtung nach einem der vorhergehenden Ansprüche, **dadurch gekennzeichnet, daß** das distale oder körperseitige Ende mindestens einer optischen Faser eine zur Ausgangsachse der Faser nicht senkrechte Ebene bildet.

14. Vorrichtung nach einem der vorhergehenden Ansprüche, **dadurch gekennzeichnet, daß** sie für die Beobachtung des Inneren des Körpers eines Lebewesens ausgelegt ist.

15. Vorrichtung nach einem der vorhergehenden Ansprüche, **dadurch gekennzeichnet, daß** sie für die Beobachtung des Inneren eines gefertigten Körpers ausgelegt ist.

## Claims

1. A device for observing the interior of a body, comprising:
- a group of fibres (300) gathered together side by side, intended to pass through an internal part of the body, comprising an outside or proximal end and an end inside of the body or distal end, said group of fibres forming an image guide the ends of which are arranged in the same order at the two inside or outside ends respectively of said group of fibres,
- means (110,115) at the proximal side for emitting an illuminating beam intended to be conveyed by the group of fibres from the outside or proximal end through a body region to observe,
- means (200) at the proximal side for scanning the outside or proximal end of the group of fibres with said illuminating beam, said scanning means being controlled for imparting successive angular deflections to the illuminating beam so that the outside or proximal end of each fibre or assembly of fibres within the group of fibres forming the image guide is successively addressed, in order to form at the inside or distal end of said fibre a secondary illuminating source at a given instant, said secondary illuminating source being moved successively from one fibre to another,
- means at the distal side for picking up a backscattered beam coming from the illuminated region and for conveying it through the group of fibres to means at the proximal side for receiving said backscattered beam,
- means for producing from the received backscattered beam an image of the illuminating region, and
- means for controlling the illuminating emission means and the scanning means, and co-operating with the image producing means,
**characterised in that** it further comprises means (L1,L2,L3,L4,L5) for forming an image conjugate plane of the illuminated region inside the body, as well as a filtration hole (600) for spatially filtering the received backscattered beam, the hole being located upstream the image production means in the conjugate image plane of the illuminating region inside the body et arranged so that only the backscattered light coming from the outside or proximal end of the addressed fibre at a given instant is treated by said image producing means.

2. Device according to claim 1, **characterised in that** each illuminated fibre is also used for conveying a return beam originating from the region of the body illuminated by it, and **in that** the scanning means comprise means (200) for imparting successive angular deflections of the illuminating beam arranged to cause a return beam emerging from this same fibre to travel along the same optical path as that of the illuminating beam entering the said fibre.

3. Device according to claim 2, **characterised in that** for each successive angular deflection, a fibre (300) corresponding to this deflection has a length such that the roundtrip propagation time of the light in the fibre (300) is equal to half the time during which the illuminating means maintain the said angular deflection, and the illuminating means (110, 115, 200) illuminate the fibre during the first half of the time interval in which the illuminating means maintain the said angular deflection.

4. Device according to any one of the preceding claims, **characterised in that** it comprises two image display devices, one adapted for presenting a view of a general region and the other a microscopic view of a more restricted region and **in that** it includes means (LM1, LM2) for simultaneously displaying the two views on the two image display devices.

5. Device according to any one of the preceding claims, **characterised in that** the illuminating means are provided for illuminating fibres (300) with a light that produces a transformation of the body substance.

6. Device according to claim 5, **characterised in that** the image display means (600, 700, 800) include means enabling a user to select, on the image displayed, a region to be treated, these means being provided for converting the indication of this region into references of fibres and for controlling the illuminating means (110, 115, 200) to illuminate the fibres corresponding to these references with the light producing the said transformation.

7. Device according to any one of the preceding claims, **characterised in that** it comprises means (110, 115, 200, 300, 400) for producing in the scanned region of the body, a point of concentration of the illuminating light rays.

8. Device according to the preceding claim 7, **characterised in that** it further includes means for adjusting the depth position of the point of concentration of the light rays as well as means for storing the images received for different settings of depth position in order to supply a three-dimensional image of the observed part of the body.

9. Device according to claims 7 and 8, **characterised in that** the means (400) for concentrating the rays are positioned at the body-side end of the image guide and **in that** the means for adjusting the depth position of the point of concentration are directly constituted by means for setting the distance of the means of concentration relative to the body-side end of the image guide.

10. Device according to one of claims 7 through 9, **characterised in that** it includes means (L1, L2, L3, 300, 200) that have an optical reception inlet (L1), these means being adapted to transmit to the image display means (600, 700, 800) only the signals obtained from return light rays coming from the point of concentration in the optical reception inlet (L1).

11. Device according to claim 10, **characterised in that** the means for concentrating the light rays and the means at reception inlet (400) are formed by a same module (400), the illuminating beam and the return beam having the same path within said module (400).

12. Device according to any one of the preceding claims, **characterised in that** it further includes means (800) for recording luminous information originating from illuminated parts of the body in the course of scanning and adapted to record, on the basis of this luminous information, information on the position of the part illuminated in the scanned region, for reconstituting an image on the basis of these two sets of data.

13. Device according to one of the preceding claims, **characterised in that** the distal end or body side of at least one optical fibre forms a plane that is not perpendicular to the outlet axis of the fibre.

14. Device according to one of the preceding claims, **characterised in that** it is adapted for observing the interior of the body of a living being.

15. Device according to one of the preceding claims, **characterised in that** it is adapted for observing the interior of a manufactured device.
